# FASCICULE DE BREVET EUROPEEN

(11) **EP 0 786 238 B1**
(45) Date de publication et mention de la délivrance du brevet: **20.03.2002**
(21) Numéro de dépôt: 97420013.1
(22) Date de dépôt: 22.01.1997
(51) Int. Cl.: A61F 2/38

(54) **Prothèse tibiale**
Tibiale Prothese
Tibial prosthesis

(30) Priorité: 23.01.1996 FR 9601012
(43) Date de publication de la demande: 30.07.1997
(73) Titulaire: TORNIER SA, 38330 Saint-Ismier (FR)
(72) Inventeur: Oudard, Jean-Loup, 38330 Saint Nazaire Les Eymes (FR); Chambat, Pierre, 69110 Sainte Foy Les Lyon (FR); Deschamps, Gérard, 71640 Givry (FR)
(74) Mandataire: Myon, Gérard Jean-Pierre

(56) Documents cités:
- EP-A- 0 001 147
- DE-A- 3 136 636
- US-A- 4 207 627
- US-A- 5 236 462

## Description

La présente invention a trait à une prothèse tibiale totale ou unicompartimentale comportant un plateau réalisé en une matière plastique frettée sur son pourtour par un anneau découpé dans de la tôle ou usiné dans la masse.

On connaît deux types de plateaux permettant de réaliser des prothèses tibiales totales ou unicompartimentales.

Les premiers sont réalisés dans une importante masse de matière plastique, mais ils ont pour inconvénient une faible rigidité et une mauvaise tenue au fluage. De plus, ce genre de plateau ne permet pas d'avoir des systèmes d'ancrage résistants.

Les seconds plus communément appelés "plateaux métal back" comportent une assise métallique rigide solidaire ou non d'une queue d'ancrage qui pénètre dans le canal médullaire de l'os recevant la prothèse tibiale. Cette assise métallique comprend des moyens de fixation pour la mise en place d'un élément en matière plastique de faible épaisseur et dont l'une des faces présente des empreintes pour recevoir les condyles du fémur. Ce type de plateau, ayant une bonne rigidité et un système d'ancrage adéquat, présente un défaut de longévité provenant du fait de la faible épaisseur de matière plastique de l'élément tibial.

Par ailleurs, DE-A-31 36 636 divulgue une prothèse comprenant un plateau en matière plastique et un support rigide encliqueté sur ce plateau. Compte tenu du mode d'assemblage, le plateau peut se déformer sous une forte charge.

C'est à ces inconvénients qu'entend plus particulièrement remédier la présente invention.

La prothèse tibiale suivant la présente invention comprend un anneau métallique et un plateau en matière plastique dont l'une des faces comporte des moyens de fixation en retrait par rapport aux bords du plateau et délimitant une ceinture plane pour permettre la mise en place de l'anneau qui assure un frettage de ladite prothèse.

En outre, l'anneau métallique comporte des dents verticales dirigées vers l'extérieur du plateau pour permettre l'ancrage de la prothèse tibiale.

Le dessin annexé, donné à titre d'exemple, pemettra de mieux comprendre l'invention, les caractéristiques qu'elle présente et les avantages qu'elle est susceptible de procurer:

Figure 1 est une vue en perspective éclatée montrant le plateau en matière plastique et l'anneau métallique de la prothèse tibiale suivant la présente invention.

Figure 2 est une vue par dessous de la prothèse tibiale illustrant le positionnement de l'anneau métallique sur le plateau.

Figure 3 est une coupe suivant III-III de figure 2 représentant la mise en place de l'anneau métallique sur le plateau.

Figures 4 et 5 sont des vues illustrant une variante de mise en place de l'anneau métallique sur le plateau.

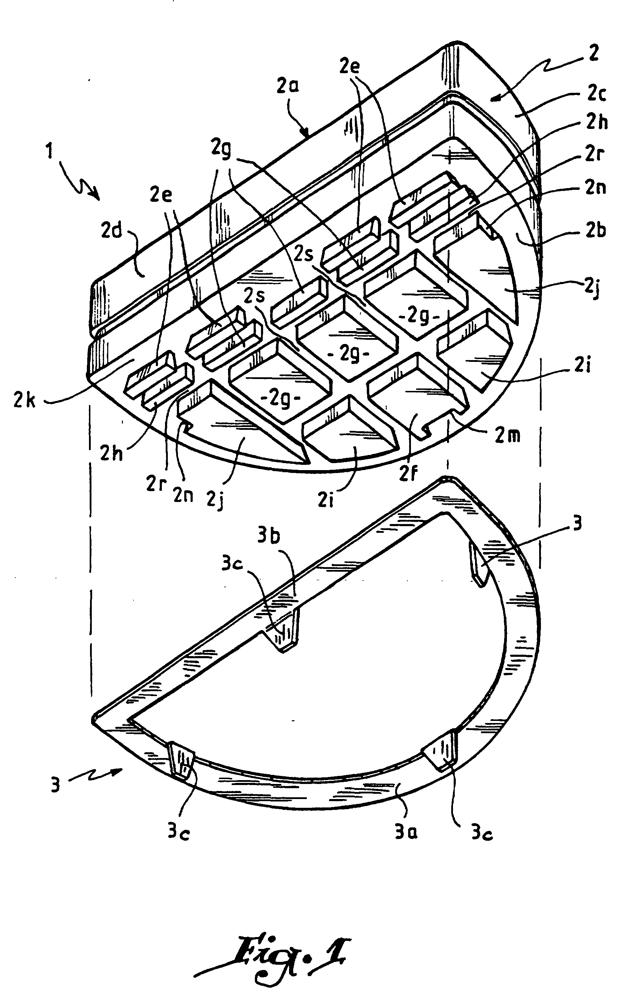

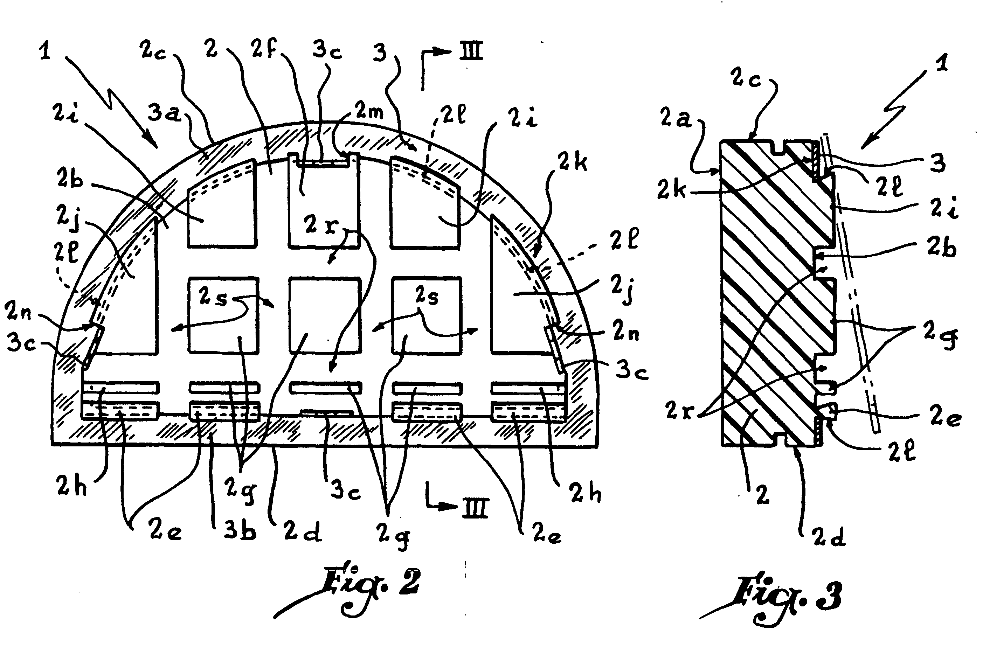

On a représenté en figure 1 à 3 une prothèse tibiale 1 unicompartimentale comportant un plateau 2 réalisé en matière plastique sur lequel vient s'encliqueter un anneau métallique 3 réalisé dans une tôle ou pièce usinée de faible épaisseur.

Le plateau 2 comporte deux faces opposées et parallèles 2a, 2b dont celle supérieure présente une ou plusieurs empreintes qui sont destinées à recevoir le ou les condyles naturels ou prothétiques du fémur. En effet, la prothèse 1 peut être soit totale, soit unicompartimentale suivant le cas opératoire.

La distance séparant les deux faces opposées parallèles 2a et 2b est suffisante pour que l'épaisseur de matière plastique soit maximale dans la partie centrale du plateau 2.

Le plateau 2 est conformé suivant une moitié de disque présentant un bord 2c à profil courbe ou en arc de cercle se prolongeant par un bord 2d à profil rectiligne.

La face 2b comporte des moyens de fixation de l'anneau métallique 3 pour constituer un frettage du plateau 2. Les moyens de fixation sont constitués par exemple de plusieurs plots en relief 2e et 2j qui sont obtenus lors de l'usinage de la face 2b. La configuration et l'agencement des plots en relief 2e à 2j peuvent être modifiés sans pour cela changer l'objet de la présente invention.

On observe que les plots périphériques en relief 2e, 2f, 2h, 2i et 2j sont en retrait par rapport aux bords 2c et 2d du plateau 2 pour constituer une ceinture plane 2k.

Les plots 2e sont disposés parallèlement au bord rectiligne 2d, tandis que les plots 2f, 2i, 2j sont conformés pour suivre le bord courbe 2c.

On constate que les plots périphériques 2e, 2f, 2h, 2i et 2j présentent chacun un pan incliné 2l qui est tourné du côté de la ceinture 2k.

Les plots périphériques 2e sont prévus avec une certaine élasticité, tandis que les plots 2f, 2i et 2j sont totalement rigides pour permettre une parfaite répartition des efforts lors de la mise en place de l'anneau métallique 3.

Les plots 2f et 2j comportent respectivement des échancrures 2m et 2n qui sont tournées du côté de la ceinture 2k du plateau 2.

Les plots en relief 2e à 2j sont séparés par des rainures horizontales 2r et verticales 2s de largeur différentes pour permettre la mise en place du ciment lors de la fixation de la prothèse 1.

L'anneau métallique 3 présente un profil semblable à celui du plateau 2. L'anneau métallique 3 comporte un pourtour courbe ou en arc de cercle 3a qui se prolonge par un second pourtour rectiligne 3b. L'anneau métallique 3 comporte des dents verticales 3c régulièrement réparties sur la périphérie interne des pourtours 3a et 3b.

Les dents verticales 3c permettent d'améliorer l'ancrage de la prothèse tibiale 1 dans deux directions perpendiculaires par rapport au plan horizontal de la face 2d du plateau 2.

On note que la déformation élastique des plots 2e permet l'encliquetage de l'anneau métallique 3 de manière que les pourtours 3a et 3b viennent se mettre en place sur la ceinture 2k et au fond des pans inclinés 2l de chaque plot périphérique 2e, 2f, 2h, 2i et 2j afin d'éviter tout retrait de l'anneau métallique 3.

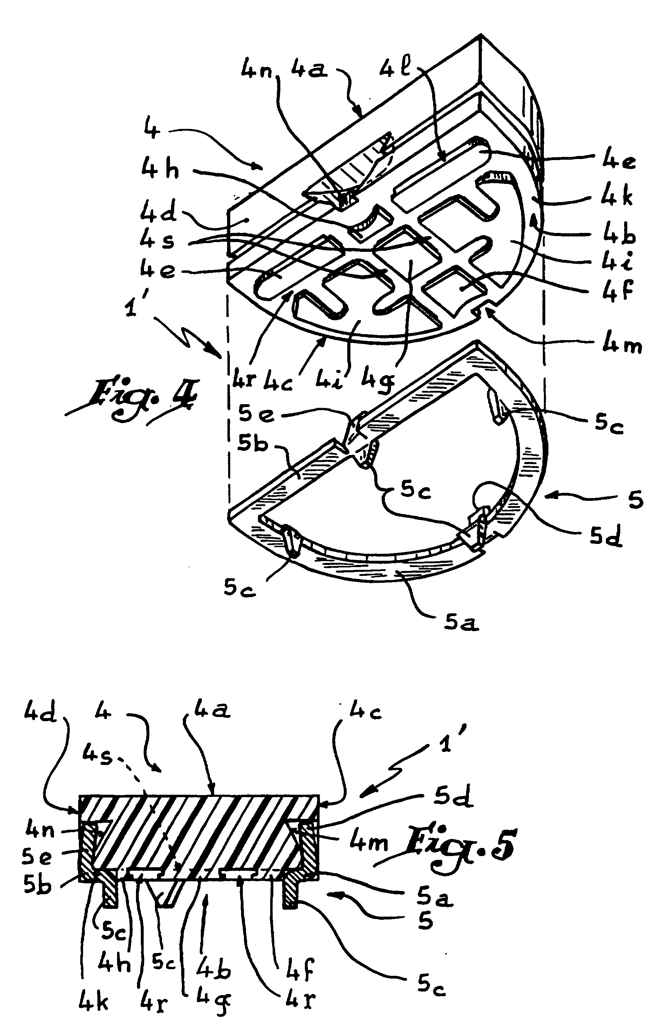

On a représenté en figure 4 et 5 une variante de la prothèse tibiale 1 référencée 1' comportant un plateau 4 réalisé en matière plastique dans lequel vient s'encliqueter un anneau métallique 5 réalisé dans une tôle ou pièce usinée de faible épaisseur.

Le plateau 4 comporte deux faces opposées parallèles 4a, 4b dont celle supérieure peut présenter une ou plusieurs empreintes qui sont destinées à recevoir le ou les condyles naturels ou prothétiques du fémur. En effet la prothèse 1' peut être soit totale, soit unicompartimentale suivant le cas opératoire.

De la même manière que pour la prothèse tibiale 1, la distance séparant les deux faces opposées parallèles 4a et 4b est suffisante pour que l'épaisseur de matière plastique soit maximale dans la partie centrale du plateau 4.

Le plateau 4 présente une forme identique à celui référencé 2, c'est-à-dire qu'il est conformé suivant une moitié de disque présentant un bord 4c à profil courbe ou en arc de cercle se prolongeant par un bord 4d à profil rectiligne.

La face 4b comporte des moyens de fixation de l'anneau métallique 5 pour constituer un frettage du plateau 4. Les moyens de fixation sont constitués par exemple de plusieurs plots en relief 4e à 4i qui sont obtenus lors de l'usinage de la face 4b. La configuration et l'agencement des plots en reliefs 4e à 4i peuvent être modifiés sans pour cela changer l'objet de la présente invention.

On observe que les plots périphériques en relief 4e, 4f, 4h et 4i sont en retrait par rapport aux bords 4c et 4d du plateau 4 pour constituer une ceinture plane 4k.

Les plots 4e et 4h sont disposés parallèlement au bord rectiligne 4d, tandis que les plots 4f et 4i sont conformés pour suivre le bord courbe 4c.

On constate que les plots périphériques 4e et 4i présentent chacun un pan incliné 4l qui est tourné du côté de la ceinture 4k. Il peut être prévu dans certains cas que les plots périphériques ne comportent pas de pans inclinés et uniquement un pan perpendiculaire à la ceinture 4k.

Le bord à profil courbe 4c comporte une échancrure 4m qui débouche partiellement sur la ceinture 4k au niveau du plot 4f. Egalement, le bord rectiligne 4d présente une échancrure 4n en face de celle 4m et qui débouche partiellement sur la ceinture 4k au niveau du plot 4h.

les plots en relief 4e à 4i sont séparés par des rainures horizontale 4r et verticale 4s de largeur différentes pour permettre la mise en place du ciment lors de la fixation de la prothèse 1'.

L'anneau métallique 5 présente un profil semblable à celui du plateau 4. La forme de l'anneau peut être quelconque, pourvu qu'il réalise un frettage du plateau 4. L'anneau métallique 5 comporte un pourtour courbe ou en arc de cercle 5a qui se prolonge par un second pourtour rectiligne 5b.

L'anneau métallique 5 comporte des dents verticales 5c régulièrement réparties sur la périphérie interne des pourtours 5a et 5b. Les dents verticales 5c permettent d'améliorer l'ancrage de la prothèse tibiale 1' dans deux directions perpendiculaires par rapport au plan horizontal de la face 4b du plateau 4.

Le pourtour 5a présente sur sa périphérie externe et au niveau d'une dent 5c une autre dent 5d dirigée dans une direction opposée à celle 5c. Egalement, le pourtour rectiligne 5b comporte sur sa périphérie externe et au niveau de la dent 5c une autre dent 5e dirigée dans une direction opposée à celle 5c.

On note que la déformation élastique des dents 5d et 5e permet l'encliquetage de l'anneau métallique 5 de manière que les pourtours 5a et 5b viennent se mettre en place sur la ceinture 4k et contre les pans 4l de chaque plot périphérique 4e et 4i afin d'éviter tout retrait de l'anneau métallique 5. En effet, les dents 5d et 5e pénètrent respectivement dans les échancrures 4n et 4m ménagées dans les bords 4c et 4d du plateau 4.

Les pourtours 3a, 5a et 3b, 5b peuvent être reliés par deux bandes parallèles non représentées améliorant la rigidité et le positionnement de l'anneau métallique 3, 5.

Lorsque l'anneau métallique 3, 5 comporte des bandes parallèles, ces dernières coopèrent avec les rainures 2s, 4s prévues à cet effet.

Il va de soi que ce type de prothèse tibiale peut être prévu pour des plateaux unicompartimentaux ou totaux recevant les condyles d'un fémur naturel ou prothétique.

On note que la configuration de l'anneau métallique 3, 5 dépend du profil donné à chaque plot en relief 2e à 2j ; 4e à 4i lors de l'usinage de la face 2b, 4b.

## Revendications

1. Prothèse tibiale comprenant un anneau métallique (3 ; 5) et un plateau (2 ; 4) en matière plastique dont la face inférieure destinée à venir en contact avec le tibia (2b ; 4b) comporte des moyens (2e-2j ; 4e-4i) de fixation en retrait par rapport aux bords (2c, 2d ; 4c, 4d) dudit plateau et délimitant une ceinture plane (2k ; 4k) pour la mise en place dudit anneau (3 ; 5) qui assure le frettage du plateau (2 ; 4).

2. Prothèse tibiale suivant la revendication 1, **caractérisée en ce que** les moyens de fixation sont constitués de plusieurs plots en reliefs (2e - 2j) dont certains (2e) sont déformables élastiquement pour l'encliquetage de l'anneau métallique (3).

3. Prothèse tibiale suivant la revendication 2, **caractérisée en ce que** certains plots (2f, 2j) comportent respectivement des échancrures (2m, 2n) tournées du côté d'une ceinture (2k) du plateau (2).

4. Prothèse tibiale suivant la revendication 1, **caractérisée en ce que** les moyens de fixation sont constitués de plusieurs plots en reliefs (4e - 4j) et de dents (5d, 5e) dudit anneau métallique (5) déformables élastiquement et qui coopèrent respectivement avec des échancrures (4m, 4n) dudit plateau pour l'encliquetage de l'anneau métallique (5) sur le plateau (4).

5. Prothèse tibiale suivant la revendication 4, **caractérisée en ce que** lesdites dents (5d, 5e) sont dirigées dans une direction opposée à celle de dents (5c) de l'anneau (5) prévues pour améliorer l'ancrage de la prothèse (1').

6. Prothèse tibiale suivant l'une des revendications 2 à 5, **caractérisée en ce que** les plots périphériques (2e, 2f, 2i - 2j ; 4e, 4f, 4i, 4j) sont disposés en retrait par rapport auxdits bords (2c, 2d ; 4c, 4d) du plateau (2 ; 4) pour constituer ladite ceinture plane (2k ; 4k).

7. Prothèse tibiale suivant la revendication 6, **caractérisée en ce que** les plots périphériques (2e, 2f, 2i - 2j ; 4e, 4i) comportent chacun un pan incliné (2l ; 4l) tourné du côté de la ceinture (2k ; 4k) pour permettre le blocage de l'anneau métallique (3, 5) sur le plateau (2 ; 4).

8. Prothèse tibiale suivant l'une des revendications 2 à 7, **caractérisée en ce que** les plots en reliefs (2e - 2j ; 4e - 4i) sont séparés par des rainures (2r, 2s ; 4r, 4s).

9. Prothèse tibiale suivant l'une des revendications 2 à 8, **caractérisée en ce que** la configuration et l'agencement des plots en relief (2e - 2j ; 4e - 4j) sur ladite face inférieure (2b ; 4b) peuvent être quelconques.

10. Prothèse tibiale suivant lune des revendications précédentes, **caractérisée en ce que** le plateau (2, 4) présente dans sa partie centrale une épaisseur de matière plastique maximale.

11. Prothèse tibiale suivant l'une des revendications précédentes, **caractérisée en ce que** l'anneau métallique (3, 5) est réalisé dans une tôle découpée de faible épaisseur comportant un pourtour courbe (3a, 5a) se prolongeant par un pourtour rectiligne (3b, 5b).

12. Prothèse tibiale suivant la revendication 11, **caractérisée en ce que** les pourtours (3a, 3b ; 5a, 5b) sont reliés par des bandes améliorant la rigidité et le positionnement de l'anneau météllique (3, 5).

13. Prothèse tibiale suivant l'une des revendications précédentes, **caractérisée en ce que** l'anneau métallique (3 ; 5) comporte des dents verticales (3c ; 5c) permettant l'ancrage du plateau (2) en matière plastique.

14. Prothèse selon l'une des revendications précédentes, **caractérisée en ce que** ledit anneau métallique (3 ; 5) présente un profil semblable à celui dudit plateau (2 ; 4).

## Patentansprüche

1. Schienbeinprothese, umfassend einen metallenen Ring (3; 5) und eine Platte (2; 4) aus Kunststoff, deren Unterseite (2b; 4b), die dazu bestimmt ist, mit dem Schienbein in Berührung zu kommen, Befestigungsmittel (2e-2j; 4e - 4i) umfasst, die im Verhältnis zu den Rändern (2c, 2d; 4c, 4d) der Platte nach hinten versetzt sind und eine ebene Einfassung (2k; 4k) zur Unterbringung des Ringes (3; 5) begrenzen, der die Versteifung der Platte (2; 4) sicherstellt.

2. Schienbeinprothese nach Anspruch 1, **dadurch gekennzeichnet, dass** die Befestigungsmittel aus mehreren erhabenen Klötzen (2e - 2j) bestehen, von denen bestimmte (2e) zum Einrasten des metallenen Rings (3) elastisch verformbar sind.

3. Schienbeinprothese nach Anspruch 2, **dadurch gekennzeichnet, dass** bestimmte Klötze (2f,2j) jeweils von der Seite der Einfassung (2k) der Platte (2) abgewandte bogenförmige Aussparungen (2m, 2n) umfassen.

4. Schienbeinprothese nach Anspruch 1, **dadurch gekennzeichnet, dass** die Befestigungseinrichtungen aus mehreren erhabenen Klötzen (4e - 4j) und Zacken (5d, 5e) des metallenen Rings (5) bestehen, die elastisch verformbar sind und jeweils mit Aussparungen (4m, 4n) der Platte zum Einrasten des metallenen Rings (5) an der Platte (4) zusammenwirken.

5. Schienbeinprothese nach Anspruch 4, **dadurch gekennzeichnet, dass** die Zacken (5d, 5e) in einer zu den Zacken (5c) des Rings (5) entgegengesetzten Richtung ausgerichtet sind, die vorgesehen sind, um die Verankerung der Prothese (1') zu verbessern.

6. Schienbeinprothese nach einem der Ansprüche 2 bis 5, **dadurch gekennzeichnet, dass** die peripheren Klötze (2e, 2f, 2i, 2j; 4e, 4f, 4i, 4j) im Verhältnis zu den Rändern (2c, 2d; 4c, 4d) der Platte (2; 4) nach hinten versetzt sind, um die ebene Einfassung (2k; 4k) auszubilden.

7. Schienbeinprothese nach Anspruch 6, **dadurch gekennzeichnet, dass** die peripheren Klötze (2e, 2f, 2i, 2j; 4e, 4i) jeweils eine geneigte Fläche (2l; 4l) aufweisen, die von der Seite der Einfassung (2k; 4k) abgewandt ist, um die Arretierung des metallenen Rings (3, 5) an der Platte (2; 4) zu ermöglichen.

8. Schienbeinprothese nach einem der Ansprüche 2 bis 7, **dadurch gekennzeichnet, dass** die erhabenen Klötze (2e - 2j; 4e - 4i) durch Rillen (2r, 2s; 4r, 4s) getrennt sind.

9. Schienbeinprothese nach einem der Ansprüche 2 bis 8, **dadurch gekennzeichnet, dass** die Konfiguration und Anordnung der erhabenen Klötze (2e - 2j; 4e - 4i) an der Unterseite (2b; 4b) beliebig sein können.

10. Schienbeinprothese nach einem der vorhergehenden Ansprüche, **dadurch gekennzeichnet, dass** die Platte (2, 4) in ihrem mittleren Teil eine maximale Kunststoffdicke aufweist.

11. Schienbeinprothese nach einem der vorhergehenden Ansprüche, **dadurch gekennzeichnet, dass** der metallene Ring (3, 5) aus einem Blech geringer Dicke ausgeschnitten ist, das einen gekrümmten äußeren Umfang (3a, 5a) umfasst, der sich in einem geradlinigen äußeren Umfang (3b, 5b) fortsetzt.

12. Schienbeinprothese nach Anspruch 11, **dadurch gekennzeichnet, dass** die äußeren Umfänge (3a, 3b; 5a, 5b) durch Leisten verbunden sind, die die Steifigkeit und die Positionierung des metallenen Rings (3, 5) verbessern.

13. Schienbeinprothese nach einem der vorhergehenden Ansprüche, **dadurch gekennzeichnet, dass** der metallene Ring (3; 5) vertikale Zacken (3c; 5c) umfasst, die die Verankerung der Platte (2) aus Kunststoff zu ermöglichen.

14. Schienbeinprothese nach einem der vorhergehenden Ansprüche, **dadurch gekennzeichnet, dass** der metallene Ring (3; 5) ein Profil ähnlich dem der Platte (2; 4) aufweist.

## Claims

1. A tibial prosthesis including a metal ring (3; 5) and a plastics material plate (2; 4) whose bottom face (2b; 4b) intended to come into contact with the tibia includes fixing means (2e-2j; 4e-4i) set back relative to the edges (2c, 2d; 4c, 4d) of said plate and delimiting a plane belt (2k; 4k) for fitting said ring (3; 5) which ensures binding of the plate (2; 4).

2. A tibial prosthesis according to claim 1, **characterized in that** the fixing means consist of a plurality of upstanding studs (2e - 2j) of which some (2e) are elastically deformable for clipping on the metal ring (3).

3. A tibial prosthesis according to claim 2, **characterized in that** some studs (2f, 2j) have respective notches (2m, 2n) facing towards a belt (2k) of the plate (2).

4. A tibial prosthesis according to claim 1, **characterized in that** the fixing means consist of a plurality of upstanding studs (4e - 4j) and teeth (5d, 5e) of said metal ring (5) that are elastically deformable and cooperate with respective notches (4m, 4n) of said plate to clip the metal ring (5) to the plate (4).

5. A tibial prosthesis according to claim 4, **characterized in that** said teeth (5d, 5e) are directed in a direction opposite that of the teeth (5c) of the ring (5) provided to improve the anchoring of the prosthesis (1').

6. A tibial prosthesis according to any of claims 2 to 5, **characterized in that** the peripheral studs (2e, 2f, 2i - 2j; 4e, 4f, 4i, 4j) are set back relative to said edges (2c, 2d; 4c, 4d) of the plate (2; 4) to constitute said plane belt (2k; 4k).

7. A tibial prosthesis according to claim 6, **characterized in that** the peripheral studs (2e, 2f, 2i - 2j; 4e, 4i) each have an inclined portion (21; 41) facing towards the belt (2k; 4k) to enable locking of the metal ring (3, 5) to the plate (2; 4).

8. A tibial prosthesis according to any of claims 2 to 7, **characterized in that** the upstanding studs (2e - 2j; 4e - 4i) are separated by grooves (2r, 2s; 4r, 4s).

9. A tibial prosthesis according to any of claims 2 to 8, **characterized in that** the upstanding studs (2e - 2j; 4e - 4j) on said bottom face (2b; 4b) can have any configuration and arrangement.

10. A tibial prosthesis according to any preceding claim, **characterized in that** the plate (2, 4) has a maximum thickness of plastics material in its central part.

11. A tibial prosthesis according to any preceding claim, **characterized in that** the metal ring (3, 5) is cut out from thin plate having a curved perimeter (3a, 5a) extended by a rectilinear perimeter (3b, 5b).

12. A tibial prosthesis according to claim 11, **characterized in that** the perimeters (3a, 3b; 5a, 5b) are joined by bands for improving the stiffness and the position of the metal ring (3, 5).

13. A tibial prosthesis according to any preceding claim, **characterized in that** the metal ring (3, 5) has vertical teeth (3c; 5c) for anchoring the plastics material plate (2).

14. A prosthesis according to any preceding claim, **characterized in that** said metal ring (3; 5) has a profile similar to that of said plate (2; 4).
